# EUROPÄISCHE PATENTSCHRIFT

(11) **EP 2 733 489 B1**
(45) Veröffentlichungstag und Bekanntmachung des Hinweises auf die Patenterteilung: **12.04.2017**
(21) Anmeldenummer: 12192740.4
(22) Anmeldetag: 15.11.2012
(51) Int. Cl.: G01N 33/28, G01N 21/35

(54) **Extraktion mehrerer Einzelgase aus einer Isolierflüssigkeit**
Extraction of several individual gases from an insulation liquid
Extraction de plusieurs gaz isolés d'un liquide d'isolation

(43) Veröffentlichungstag der Anmeldung: 21.05.2014
(73) Patentinhaber: Siemens Aktiengesellschaft, 80333 München (DE)
(72) Erfinder: Atanasova-Höhlein, Ivanka, 90762 Fürth (DE); Buchgraber, Gerhard, 8182 Puch bei Weiz (AT); Kuffel, Maik, 96052 Bamberg (DE); Rehorek, Christian, 90522 Oberasbach (DE); Zafeiris, Konstantinos, 90419 Nürnberg (DE)

(56) Entgegenhaltungen:
- WO-A1-98/36265
- WO-A1-2010/127759
- DD-A1- 218 465
- DE-A1- 3 227 840
- JP-A- H06 148 068
- US-B1- 6 526 805
- DUVAL M: "The duval triangle for load tap changers, non-mineral oils and low temperature faults in transformers", IEEE ELECTRICAL INSULATION MAGAZINE, IEEE SERVICE CENTER, NEW YORK, NY, US, Bd. 11, Nr. 6, November 2008 (2008-11), Seiten 22-29, XP011266847, ISSN: 0883-7554, DOI: 10.1109/MEI.2008.4665347
- TSUKIOKA H ET AL: "Niew Apparatus for Detecting Transformer Faults", IEEE TRANSACTIONS ON ELECTRICAL INSULATION, IEEE, US, Bd. EI-20, Nr. 2, April 1986 (1986-04), Seiten 221-229, XP011177581, ISSN: 0018-9367
- JOSEPH J KELLY: "Transformer Fault Diagnosis by Dissolved-Gas Analysis", IEEE TRANSACTIONS ON INDUSTRY APPLICATIONS, IEEE SERVICE CENTER, PISCATAWAY, NJ, US, Bd. IA-16, Nr. 6, November 1980 (1980-11), Seiten 777-782, XP001337943, ISSN: 0093-9994
- JAKOB F ET AL: "Use of gas concentration ratios to interpret LTC & OCB dissolved gas data", PROCEEDINGS OF THE ELECTRICAL INSULATION CONFERENCE AND ELECTRICAL MANUFACTURING AND COIL WINDING CONFERENCE. (COMBINED CONFERENCE). INDINAPOLIS, IN, SEPT. 23 - 25, 2003; [PROCEEDINGS OF THE ELECTRICAL ELECTRONICS INSULATION CONFERENCE AND ELECTRICAL, 23. September 2003 (2003-09-23), Seiten 301-304, XP010670664, DOI: 10.1109/EICEMC.2003.1247901 ISBN: 978-0-7803-7935-0

## Beschreibung

Die Erfindung betrifft ein Messverfahren für einen Transformator, bei dem mehrere Einzelgase aus einer Isolierflüssigkeit extrahiert und mittels einer Auswerteeinheit aus einem Messsignal und/oder aus einem daraus abgeleiteten Wert mindestens ein Gasparameter bestimmt wird. Die Erfindung betrifft ferner eine Isolierflüssigkeits-Bestimmvorrichtung für einen Transformator zum Durchführen des Verfahrens mit einem Extraktor und einer Auswerteeinheit, wobei der Extraktor eine Isolierflüssigkeit- oder Ölseite und eine Gasseite aufweist und zum Extrahieren mehrerer Einzelgase aus der Isolierflüssigkeit- oder Ölseite in die Gasseite eingerichtet ist und wobei die Auswerteeinheit ausgelegt ist zum Bestimmen mindestens eines Gasparameters der mehreren Einzelgase. Die Erfindung ist insbesondere anwendbar auf eine Extraktion mehrerer Einzelgase aus Transformatorenöl.

Sogenannte Schadgassensoren bestehen im Prinzip aus einer Extraktionseinrichtung mit Ölkreislauf und einer Messeinrichtung mit einem Gaskreislauf. Sowohl im Ölkreis als auch im Gaskreis werden Pumpen für eine Zirkulation des Isolieröls bzw. des extrahierten Gasgemischs benötigt. Dabei notwendige Baugruppen wie Ventile, Pumpen und Steuerelektronik sind wie alle technischen Systeme mit einer Fehlerrate behaftet und führen zu einer unerwünscht hohen Ausfallsrate und hohem Wartungsbedarf. Eine technische Lebensdauer solcher Sensoren liegt damit weit unter der technischen Lebensdauer der Primärgeräte, welche der Sensor zu überwachen hat.

Im Isolieröl gelöste Schadgase werden entweder über eine Membran oder eine freie Öloberfläche (Headspace) in den gasförmigen Zustand gebracht und dann mit Hilfe einer Pumpe in einen Detektor zur Messung geleitet.

Unter anderem DIN EN 60599 beschreibt in Betrieb befindliche, mit Mineralöl imprägnierte elektrische Geräte. Beschrieben wird darin insbesondere das Messen von im Öl enthaltenen Gasen und damit als solches die Bestimmung von Basis-Gasquotienten Acetylen (C₂H₂) / Ethen (C₂H₄), Methan (CH₄) / Wasserstoff (H₂) bzw. Ethen (C₂H₄) / Ethan (C₂H₆), um einen Ölzustand zu prüfen bzw. auf Fehler im Betrieb des elektrischen Geräts zu schließen. Dadurch lässt sich auf thermische und elektrische fehlerhafte Betriebszustände schließen. Durch eine Untersuchung des Öls auf einen Gehalt an Kohlenmonoxid, Kohlendioxid und Wasser lässt sich auf eine Zersetzung einer Zelluloseisolierung des elektrischen Geräts schließen.

On-line-Monitoring an Transformatoren wird in Hinsicht auf eine Energieentwicklung (SmartGrid, Offshore) immer wichtiger. Gas-in-Öl-Überwachungssysteme können Fehler erkennen und Trends kontinuierlich aufzeichnen. Derzeit existieren zwei Hauptrichtungen der On-Line-Überwachungs-Philosophie: gemäß einem Ansatz gibt es einfache Systeme, die nur ein Fehlergas bestimmen. Ein-Gas-Überwachungssysteme weisen jedoch nachteiligerweise keine Möglichkeit einer Diagnose auf, wodurch die Anwendungsmöglichkeit eingeschränkt ist. Diese Systeme werden hauptsächlich als Frühwarnsysteme angewendet. Außerdem gibt es Vollsysteme, die eine Gas-in-Öl-Analyse auf der Basis einer Auswertung mehrerer Einzelgase vollständig ersetzen können. Nachteilig dabei ist, dass Vollsysteme teuer in der Anschaffung und kompliziert in Anwendung und Wartung sind.

Bekannt ist zur Gasanalyse allgemein die Verwendung eines Gaschromatographen, mit dem unter Verwendung eines Trägergases aus dem Öl extrahiertes oder aus dem Öl entgastes Gas zur Analyse des Gases verbrannt wird.

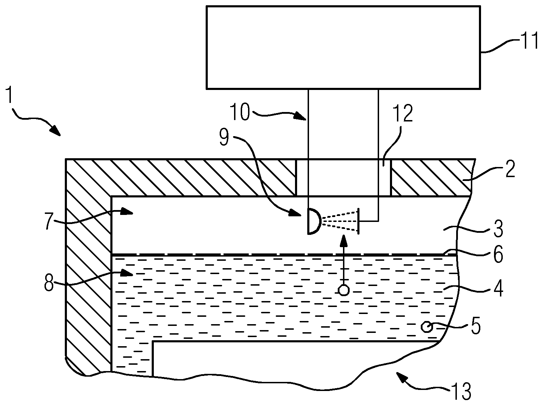

Aus der DD 218 465 A1 ist ein Verfahren zur Überwachung ölisolierter Hochspannungsgeräte, insbesondere Öltransformatoren, bekannt. Es ist nirgends in D1 offenbart oder darauf hingewiesen, gezielt einen Gasparameter zu bestimmen, der für eine Gruppe ungesättigter Kohlenwasserstoffe und/oder für eine Gruppe gesättigter Kohlenwasserstoffe gebildet wird. Dabei ist es zunächst unerheblich, ob die in D1 erwähnte Vorrichtung solche Gruppen messen könnte oder nicht, zumal in D1, Fig.2, nur Gruppenmessungen für gemischte ungesättigte und gesättigte Kohlenwasserstoffe offenbart sind.

Auch die DE3227840 A1 offenbart ein Verfahren zur Überwachung mit Öl gefüllter elektrischer Einrichtungen, wobei Gasparameter von einzelnen Gasen, unter anderem gesättigter und ungesättigter Kohlenwasserstoffe, gemessen werden und aus Quotienten dieser Gasparameter auf bestimmte Betriebszustände der Einrichtungen geschlossen wird. Es wird jedoch nicht offenbart, einen Gasparameter für eine Gruppe gesättigter und/oder eine Gruppe ungesättigter Kohlenwasserstoffe.

Die WO 2010/127759 A1 betrifft ein Verfahren zur quantitativen Analyse von Gasen als Monitoring nur für Laststufenschalter. Die allgemeine Idee besteht darin, bestimmte charakteristische Gase, die eine indirekte nachfolgende Zuordnung und ein Maß für Alterungserscheinungen, übermäßige Entladung und/oder Erwärmung am Stufenschalter ermöglichen, auszuwählen, diese charakteristischen Gase, die sich während des Betriebes des Stufenschalters in dessen Isolieröl bilden, in bestimmten Zeitabständen zu messen, aus den gemessenen Werten der definierten Gase aussagekräftige Quotienten zu bilden, die direkte Rückschlüsse auf Alterungserscheinungen, übermäßige Entladung und/oder Erwärmung zulassen, aus einem Abgleich des aktuellen ermittelten Wertes des jeweiligen Quotienten mit dem zuletzt ermittelten Wert des selben Quotienten Tendenzen aufzuzeigen und daraus Warnungen für Alterungserscheinungen, übermäßige Entladung und/oder Erwärmung abzuleiten, wenn sich die entsprechenden Quotienten im Lauf der Zeit oder im Verlauf der absolvierten Schaltungen tendenziell signifikant verändern.

Es ist die **Aufgabe** der vorliegenden Erfindung, die Nachteile des Standes der Technik zumindest teilweise zu überwinden. Eine besondere Aufgabe besteht darin, eine Isolierflüssigkeits-Bestimmvorrichtung für einen Transformator mit einem Extraktor zu verbessern bzw. ein Messverfahren für einen Transformator, bei dem mindestens ein Gas aus einer Isolierflüssigkeit, insbesondere Öl, extrahiert und mittels einer Auswerteeinheit mindestens ein Gasparameter bestimmt wird, zu verbessern. Insbesondere soll eine sichere Bestimmung von Gasen mit einfachem Aufwand bei einfacher Konstruktion ermöglicht werden.

Diese Aufgabe wird gemäß den Merkmalen der unabhängigen Ansprüche gelöst. Bevorzugte Ausführungsformen sind insbesondere den abhängigen Ansprüchen entnehmbar.

Eine Lösung besteht demgemäß in einem Verfahren zum Bestimmen mindestens eines Gasparameters für einen Transformator, bei dem mehrere Einzelgase aus einer Isolierflüssigkeit extrahiert und mittels einer Auswerteeinheit aus einem Messsignal und/oder einem daraus abgeleiteten Wert der mindestens eine Gasparameter bestimmt wird, wobei der mindestens eine Gasparameter mindestens einen Gas-Summenparameter, der für eine Gruppe ungesättigter Kohlenwasserstoffe gebildet wird, und/oder mindestens einen Gas-Summenparameter, der für eine Gruppe gesättigter Kohlenwasserstoffe gebildet wird, umfasst. Mit anderen Worten wird mindestens ein solcher Gas-Summenparameter anstelle eines Einzelgasparameters bestimmt und kann entsprechend zur Diagnose, Fehlersuche usw. des elektrischen Antriebsmittels verwendet werden.

Unter einem Gasparameter kann insbesondere ein zu einem Einzelgas oder zu einem Gasgemisch gehöriger Parameter verstanden werden, z.B. ein bestimmtes Messsignal und/oder mindestens ein daraus abgeleiteter Wert wie eine Gasmenge und/oder eine Konzentration. Unter einem Einzelgasparameter kann insbesondere ein zu einem bestimmten Einzelgas gehöriger Parameter verstanden werden. Unter einem Gas-Summenparameter kann insbesondere ein zu einem bestimmten Gasgemisch gehöriger Parameter verstanden werden.

Bereitgestellt wird damit eine vereinfachte Auswertung von Gasparametern. Insbesondere ergibt sich der Vorteil, dass sich ein zur Diagnose sinnvoll nutzbarer Gasparameter selbst dann noch mit einfachen Mitteln in guter Genauigkeit bereitstellen lässt, wenn eine Bestimmung eines Einzelgasparameters schwierig ist, z.B. aufgrund einer vergleichsweise geringen Konzentration eines Einzelgases oder einer Überlagerung von Messsignalen von Einzelgasen. Das Verfahren ist insbesondere auch einsetzbar in Verbindung mit Gasparametern und Gasbestandteilen, welche normgerecht zur Bestimmung von Fehlern einsetzbar sind. Insbesondere ermöglichen diese Parameter eine Bestimmung elektrischer und thermischer Fehler.

Der Gas-Summenparameter, der für eine Summe oder Gruppe ungesättigter Kohlenwasserstoffe gebildet wird, und der Summenparameter, der für eine Summe oder Gruppe gesättigter Kohlenwasserstoffe gebildet wird, weisen den Vorteil auf, dass diese besonders einfach zusammen bestimmt bzw. ausgewertet werden können.

Jedoch sind auch Mischformen möglich, z.B. dass der Gas-Summenparameter für eine Summe oder Gruppe gesättigter und ungesättigter Kohlenwasserstoffe gebildet wird. Diese können besonders einfach zusammen bestimmt bzw. ausgewertet werden.

Es ist eine Ausgestaltung, dass zur Bestimmung eines thermischen Fehlers ein Verhältnis aus einem Gas-Summenparameter ungesättigter Kohlenwasserstoffe und aus einem Gas-Summenparameter gesättigter Kohlenwasserstoffe gebildet wird.

Noch eine Ausgestaltung besteht darin, dass zusätzlich mindestens ein Einzelgasparameter bestimmt wird, insbesondere Kohlendioxid (CO₂), Kohlenmonoxid (CO), Ethin bzw. Acetylen (C₂H₂), Wasserstoff (H₂), Ethen bzw. Ethylen (C₂H₄), Methan (CH₄) und/oder Ethan (C₂H₆).

Es ist ferner eine Ausgestaltung, dass zur Bestimmung eines thermischen Fehlers ein Verhältnis aus einem Gas-Summenparameter gesättigter Kohlenwasserstoffe und einem Einzelgasparameter gebildet wird.

Noch eine Ausgestaltung besteht darin, dass ein Quotient oder Verhältnis von Gasparametern bestimmt wird, wobei mindestens eine Größe des Verhältnisses ein Gas-Summenparameter ist. Das Verhältnis kann beispielsweise dazu verwendet werden, um eine oder mehrere Auswertungen, Diagnosen und/oder Aktionen mit noch höherem Aussagewert durchzuführen als z.B. bei einer Schwellwertbetrachtung absoluter Größen. Das Verhältnis mag beispielsweise ein Verhältnis von Einzelgasparametern und Gas-Summenparametern und/oder ein Verhältnis von mehreren Gas-Summenparametern umfassen.

Es ist eine Weiterbildung davon, dass ein Verhältnis aus einem Gas-Summenparameter ungesättigter Kohlenwasserstoffe und aus einem Gas-Summenparameter gesättigter Kohlenwasserstoffe gebildet wird, insbesondere zur Bestimmung eines thermischen Fehlers. Beispielsweise kann dazu ein Verhältnis bestimmt werden aus Gas-Summenparametern der Form (ΣCₙH₂ₙ / ΣCₙH₂ₙ₊₂), insbesondere umfassend C₂H₄ und C₂H₆ (n=2). Mit anderen Worten mögen insbesondere zueinander verschiedene Gas-Summenparameter bestimmt werden, welche einerseits aus der Gruppe der ungesättigten Kohlenwasserstoffe und andererseits aus der Gruppe der gesättigten Kohlenwasserstoffe genommen werden.

Eine Weiterbildung dazu ist, dass zusätzlich ein Verhältnis aus einem Gas-Summenparameter gesättigter Kohlenwasserstoffe und einem Einzelgasparameter, z.B. Kohlenmonoxid, gebildet, insbesondere ausgewertet, wird, insbesondere zur Bestimmung eines thermischen Fehlers. Beispielhaft kann ein Quotient der Form (ΣCₙH₂ₙ₊₂ / CO) bestimmt werden. Bestimmbar sind dadurch insbesondere thermische Fehler von mit Mineralöl imprägnierten elektrischen Geräten, insbesondere unter Einsatz einer Infrarot-Sensorik. Insbesondere wird Kohlenmonoxid nicht lediglich zur Bestimmung eines Zelluloseabbaus verwendet.

Es ist auch eine Weiterbildung, dass ein Verhältnis aus Acetylen und einem Gas-Summenparameter gesättigter Kohlenwasserstoffe gebildet und ausgewertet wird, insbesondere zur Bestimmung eines elektrischen Fehlers. Dazu wird insbesondere ein Quotient der Form (C₂H₂ / ΣCₙH₂ₙ₊₂), insbesondere umfassend C₂H₆ (n=2), verwendet.

Eine weitere Weiterbildung dazu besteht darin, dass zusätzlich ein Gas-Summenparameter ungesättigter Kohlenwasserstoffe und ein Quotient aus Acetylen und einem Gas-Summenparameter gesättigter Kohlenwasserstoffe ausgewertet werden, insbesondere zur Bestimmung eines elektrischen Fehlers. Dies ist insbesondere realisierbar in der Form (ΣCₙH₂ₙ + C₂H₂ / ΣC₂H₂ₙ₊₂). Bestimmbar sind somit auch elektrische Fehler von mit Mineralöl imprägnierten elektrischen Geräten, speziell unter Einsatz einer Infrarot-Sensorik.

Noch eine weitere Weiterbildung besteht darin, dass ein Quotient aus Kohlendioxid und Kohlenmonoxid gebildet und ausgewertet wird zur Bestimmung eines Zustands einer festen Isolierung, insbesondere eines Zelluloseabbaus. Dies erfolgt insbesondere in der Form (CO₂ / CO).

Eine Ausgestaltung besteht darin, dass zum Bestimmen des mindestens einen Gas-Summenparameters eine Infrarot-Messeinrichtung mit mindestens einem Infrarotsensor verwendet wird. Eine Infrarot-Messeinrichtung ermöglicht einen einfachen Aufbau und dadurch eine einfache und ausreichend zuverlässige Bestimmung von Gas-Summenparametern.

Auch ist es eine Ausgestaltung, dass als die Isolierflüssigkeit Öl verwendet wird. Das Antriebsmittel mag insbesondere ein Transformator sein, dessen Isolierflüssigkeit dann insbesondere Transformatorenöl. Insbesondere ist eine derartige Isolierflüssigkeit nicht nur zum Isolieren, sondern zusätzlich oder alternativ auch zum Kühlen einsetzbar.

Eine weitere Lösung der Aufgabe besteht in der Ausgestaltung einer Isolierflüssigkeits-Bestimmvorrichtung für ein einen Transformator, welche zum Durchführen eines solchen Verfahrens ausgestaltet ist. Die Isolierflüssigkeits-Bestimmvorrichtung mag dazu analog zu dem Verfahren ausgestaltet sein und auch die gleichen Vorteile aufweisen.

Die Isolierflüssigkeits- Bestimmvorrichtung ist mit einem Extraktor und einer Auswerteeinheit ausgestattet, wobei der Extraktor eine Isolierflüssigkeitsseite und eine Gasseite aufweist und zum Extrahieren von Gas aus der Isolierflüssigkeitsseite in die Gasseite eingerichtet ist, die Gasseite des Extraktors mindestens eine Infrarot-Messeinrichtung mit mindestens einem Infrarotsensor zum Bestimmen mindestens des Gases in der Gasseite aufweist und wobei und wobei die Auswerteeinheit ausgelegt ist zum Bestimmen mindestens eines Gasparameters des mindestens einen extrahierten Gases, wobei die Isolierflüssigkeits- Bestimmvorrichtung eine Auswerteeinheit aufweist, wobei die Auswerteeinheit ausgelegt ist zum Bestimmen des mindestens einen Gas-Summenparameters. Dies ermöglicht einen besonders einfachen Aufbau der Anordnung bei zugleich zuverlässiger Bestimmung des mindestens einen Gasparameters.

Insbesondere kann bei der Infrarotmessung ein an der Gasseite befindlicher Gassammelraum direkt als Messzelle verwendet werden. Dadurch wird bei einfacherem konstruktivem Aufwand eine trotzdem insbesondere qualitativ und quantitativ zuverlässige Gasbestimmung ermöglicht. Insbesondere kann so die Anzahl der notwendigen Komponenten zur Gasanalyse wesentlich reduziert werden. Speziell kann eine Führung von Leitungen, insbesondere Gasleitungen, in den Gassammelraum vermieden werden, wie sie beispielsweise bei einem Gaschromatographen benötigt werden. Daraus folgen eine Reduktion der Fehleranfälligkeit und damit eine Steigerung der Zuverlässigkeit und der technischen Lebensdauer des Systems. Außerdem wird ein Wartungsaufwand des Systems aufgrund der Einfachheit der Komponenten gesenkt.

Auch ist es eine Ausgestaltung, dass die Auswerteeinheit einen Prozessor zum Durchführen eines Analysealgorithmus aufweist. Neben insbesondere einer Aufbereitung von Messwerten und einer Auswertung kann durch einen solchen Prozessor auch eine gezielte Ansteuerung von Komponenten der Vorrichtung, insbesondere eine gezielte Ansteuerung der Messeinrichtung mit mindestens einer Lichtquelle und mindestens einem Detektor umgesetzt werden.

Es ist eine Weiterbildung, dass der Analysealgorithmus ausgelegt ist, die Gasparameter aus den gemessenen Gasen zu bestimmen und auszuwerten. Insbesondere ist der Analysealgorithmus ausgelegt, mit dem bestimmten Gas-Summenparameter eine Verhältnis- bzw. Quotientenbildung mit anderen Gas-Summenparametern und/oder Einzelgasparametern durchzuführen.

In den Gassammelraum sind Komponenten der Infrarot-Messeinrichtung, insbesondere eine Infrarot-Lichtquelle und ein Infrarot-Sensor einsetzbar, ohne in direkter Berührung mit dem Öl zu stehen. Entsprechend ist es eine weitere ausgestaltende Weiterbildung, dass die Infrarot-Messeinrichtung innerhalb eines Gassammelraums des Extraktors angeordnet ist.

Alternativ mag der Gassammelraum ein oder mehrere für Infrarotlicht durchlässige Fenster aufweisen, so dass die infrarot-Lichtquelle und/oder der Infrarot-Sensor auch außerhalb des Gassammelraums angeordnet sein können. Dadurch kann auf Zuleitungen aller Art vollständig verzichtet werden.

Auch ist es eine Weiterbildung, dass die Infrarot-Messeinrichtung Bestandteil eines Infrarot-Spektrometers ist. Eine weitere Weiterbildung besteht darin, dass die Infrarot-Messeinrichtung Bestandteil eines Infrarot-Absorptionsspektrometers ist. Mit anderen Worten können vom Grundaufbau her bekannte Infrarot-Spektrometer oder Infrarot-Absorptionsspektrometer verwendet werden, deren Messzelle nun insbesondere dem Gassammelraum entsprechen mag oder alternativ gastechnisch mit dem Gassammelraum verbunden ist.

Es ist noch eine Weiterbildung, dass die Infrarot-Messeinrichtung für mehrere unterschiedliche Frequenzbereiche sensitiv ist. Dadurch können unterschiedliche Einzelgase und/oder Gasgemische genauer detektiert bzw. bestimmt werden. Gemäß einer Weiterbildung ist vorgesehen, dass die Gasseite dazu insbesondere eine Lichtquelle oder mehrere Lichtquellen, insbesondere Infrarot-Leuchtdioden der Infrarot-Messeinrichtung aufweist, die auf unterschiedlichen Frequenzen oder Frequenzbändern ausstrahlen. Auch kann gemäß einer Weiterbildung vorgesehen sein, dass die Gasseite einen Sensor bzw. Detektor oder mehrere Sensoren bzw. Detektoren der Infrarot-Messeinrichtung aufweist, welche für unterschiedliche Frequenzen oder Frequenzbänder sensitiv sind. Die verschiedenen Lichtquellen bzw. Detektoren können je nach Ausgestaltung insbesondere einander überlappende oder nicht überlappende Sensitivitätsbereiche aufweisen.

Insbesondere ist es eine Weiterbildung, dass die mindestens eine Infrarot-Messeinrichtung oder eine dieser nachgeschaltete Auswerteeinheit zum Detektieren gesättigter Kohlenwasserstoffe ausgebildet ist. Auch ist es eine Weiterbildung, dass die mindestens eine Infrarot-Messeinrichtung oder eine dieser nachgeschaltete Auswerteeinheit zum Detektieren ungesättigter Kohlenwasserstoffe ausgebildet ist.

Bereitgestellt wird insbesondere ein modular aufgebautes, preiswertes Gas-in-Öl-System zur Analyse gelöster und freier Gase (DGA), welches sowohl online als auch offline benutzt werden kann. Insbesondere besteht das System aus dem Verfahrensschritt der Extraktion der gelösten Gase aus der Isolierflüssigkeit bzw. Öl, dem Verfahrensschritt der Detektion von Gas-Summenparametern und gegebenenfalls Einzelgasen mittels Infrarot-Detektoren und einem Auswertealgorithmus, basierend auf der Auswertung von Gas-Summenparametern und gegebenenfalls Einzel-Gasparametern, Anstiegswerten und/oder Quotienten bzw. Verhältnissen davon. Das Einführen weiterer Detektoren ist möglich.

In der folgenden Figur wird die Erfindung anhand eines Ausführungsbeispiels schematisch genauer beschrieben. Die Figur zeigt in Seitenansicht teilweise geschnitten Komponenten einer Isolierflüssigkeits-Bestimmvorrichtung in Form einer Ölmessvorrichtung für ein elektrisches Antriebsmittel in Form eines Transformators mit einem Extraktor und einer Infrarot-Messeanordnung.

Die **Fig.** zeigt einen Ausschnitt eines Extraktors 1 eines Transformators 13. In einem Gehäuse 2 des Extraktors 1 befinden sich ein Gassammelraum 3 und ein Bereich mit Öl 4, welches insbesondere als Schmiermittel und/oder Isolierflüssigkeit verwendet wird.

Eine durch das Gehäuse 2 führende Membran 6 trennt eine Gasseite 7 mit dem Gassammelraum 3 und eine als Isolierflüssigkeitsseite dienende Ölseite 8 mit dem Bereich mit dem Öl 4 voneinander. Gas 5, welches sich auf der Ölseite 8 im Öl 4 befindet und mehrere Einzelgase oder Gaskomponenten aufweist, tritt durch die Membran 6 in die Gasseite 7 über und sammelt sich im Gassammelraum 3.

In dem Gassammelraum 3 ist eine Messkomponente einer Infrarot-Messeinrichtung 9 angeordnet. Der Gassammelraum 3 dient also auch als eine Messzelle. Die beispielhafte Messkomponente besteht aus einer Infrarot-Lichtquelle und einem davon beabstandeten Infrarot-Sensor bzw. -Detektor. Zwischen der Infrarot-Lichtquelle und dem Infrarot-Detektor befindet sich ein Zwischenraum, in den das in den Gassammelraum 3 eingetretene Gas 5 gelangt und dort ausgemessen wird.

Über elektrische Leitungen 10 sind die Komponenten mit einer Auswerteeinheit 11 verbunden. Insbesondere ist die Auswerteeinheit 11 als eigenständiger Prozessor ausgestaltet oder Bestandteil eines übergeordneten Vorrichtungsprozessors, welcher auch zur Steuerung anderer Funktionalitäten der Vorrichtung ausgelegt ist. Eine Öffnung 12 zum Durchführen der elektrischen Leitungen 10 kann insbesondere gasdicht verschlossen sein. Die Öffnung 12 kann aber auch offen sein oder mit einer Leitung zum Abführen gesammelten Gases verbunden sein.

Die Auswerteeinheit 11 ist hier dazu ausgelegt, zumindest aus von der Infrarot-Messeinrichtung 9 gemessenen Messwerten mindestens einen Gas-Summenparameter zu bestimmen, z.B. eine Konzentration. Insbesondere ist die Auswerteeinheit 11 ausgelegt, im Rahmen einer Auswertung zur Bestimmung von Fehlern usw. des Transformators 13 Quotienten von Gas-Summenparametern zu bilden und ggf. auszuwerten.

Für die Quotientenbildung oder Verhältnisbildung werden insbesondere gesättigte gegenüber ungesättigten Kohlenwasserstoffen bzw. ungesättigte gegenüber gesättigten Kohlenwasserstoffen betrachtet. Zur Bestimmung eines thermischen Fehlers wird insbesondere ein Quotient aus einer Gruppe ungesättigter Kohlenwasserstoffe zu einer Gruppe gesättigter Kohlenwasserstoffe betrachtet. Zur Bestimmung eines thermischen Fehlers kann zusätzlich auch eine Quotientenbildung aus einer Gruppe gesättigter Kohlenwasserstoffe zu Kohlenmonoxid betrachtet werden.

Zur Bestimmung eines elektrischen Fehlers kann insbesondere ein Quotient aus Acetylen zu einer Gruppe gesättigter Kohlenwasserstoffe betrachtet werden. Zur zusätzlichen Bestimmung eines elektrischen Fehlers mag auch eine Gruppe ungesättigter Kohlenwasserstoffe zuzüglich Acetylen im Verhältnis zu einer Gruppe gesättigter Kohlenwasserstoffen betrachtet werden.

Zur Bestimmung eines Zelluloseabbaus wird insbesondere ein Quotient aus Kohlendioxid zu Kohlenmonoxid betrachtet.

Über- bzw. Unterschreiten die Gas-Summenparameter und/oder Verhältnisse bzw. Quotienten davon bestimmte Schwellwerte, so dient dies z.B. als Warnung oder Hinweis auf einen möglichen Fehler oder Wartungsfall. Gegebenenfalls kann die Auswerteeinheit 11 auch dazu ausgelegt sein, neben der Ausgabe von Warnungen aktiv in einen laufenden Prozess einzugreifen, z.B. in den Betrieb des Transformators 13.

Eine Diagnoseausgabe seitens der Auswerteeinheit 11 erfolgt insbesondere dann, wenn bestimmte Mindestwerte erreicht werden. Beispielsweise kann als Mindestwert für Kohlenmonoxid ≥ 20 ppm angesetzt werden. Als Bedingung für eine Ausgabe eines Hinweises, einer Warnung usw. im Fall eines Gas-Summenwerts ungesättigter Kohlenwasserstoffe kann z.B. als Bedingung ≥ 10 ppm angesetzt werden.

Selbstverständlich ist die vorliegende Erfindung nicht auf das gezeigte Ausführungsbeispiel beschränkt.

Anstelle eines Gehäuses 2 des Extraktors 1, welches auch Komponenten des elektrischen Antriebsmittels 13 aufnimmt, sind insbesondere auch Anordnungen möglich, bei denen der Extraktor 1 ein eigenständiges Gehäuse 2 hat, welches über Leitungen, insbesondere Ölleitungen in einen Ölkreislauf auf der Ölseite 8 eingebunden ist. Solche Ölleitungen führen dann aus einem Innenraum eines elektrischen Antriebsmittels, beispielsweise eines Transformators, in das Gehäuse des Extraktors und insbesondere aus dem Gehäuse des Extraktors wieder zurück in den Innenraum des elektrischen Antriebsmittels.

Auch braucht eine Trennung der Ölseite 8 von der Gasseite 7 mittels der Membran 6 nicht zwingend in der skizzierten Art und Weise erfolgen. Ggf. kann die Membran ganz entfallen, wenn z.B. Gas aus einer offenen Öloberfläche in einen darüber liegenden Gassammelraum austritt. Wichtig für die insbesondere erste Ausgestaltung ist insbesondere, dass der Extraktor 1 die Gasseite 7 aufweist, in welcher z.B. Komponenten der Infrarot-Messeinrichtung 9 angeordnet sind, so dass in der Gasseite 7 befindliche Gase bestimmbar und mittels der Auswerteeinheit analysierbar sind.

Anstelle lediglich einer Infrarot-Leuchteinrichtung, insbesondere Infrarot-Leuchtdiode und eines Infrarot-Sensors bzw. Infrarot-Detektors können auch zusätzliche Komponenten der Infrarot-Messeinrichtung 11 innerhalb der Gasseite 7 bzw. des Gassammelraums 3 angeordnet sein. Insbesondere kann auch die Auswerteeinheit 11 ganz oder teilweise innerhalb der Gasseite 7 angeordnet sein. Alternativ mag die Infrarot-Messeinrichtung 9 auch ganz außerhalb des Gassammelraums 3 angeordnet sein.

Als weitere Auswertekriterien, insbesondere zur Quotientenbildung können auch weitere Einzelgase oder summierte Gase bzw. Moleküle mittels insbesondere der Infrarot-Messeinrichtung 9 zur Auswertung herangezogen werden.

Die Verwendung eines zusätzlichen Sensors, z.B. Wasserstoffsensors, kann zur weiteren Präzisierung der Diagnose eingesetzt werden.

## Patentansprüche

1. Verfahren zum Bestimmen mindestens eines Gasparameters für einen Transformator, bei dem mehrere Einzelgase (5) aus einer Isolierflüssigkeit (4) extrahiert und mittels einer Auswerteeinheit (11) aus einem Messsignal und/oder aus einem daraus abgeleiteten Wert der mindestens eine Gasparameter bestimmt wird,
**dadurch gekennzeichnet, dass** der mindestens eine Gasparameter mindestens einen Gas-Summenparameter, der für eine Gruppe ungesättigter Kohlenwasserstoffe gebildet wird, und/oder mindestens einen Gas-Summenparameter, der für eine Gruppe gesättigter Kohlenwasserstoffe gebildet wird, umfasst.

2. Verfahren nach Anspruch 1, wobei zur Bestimmung eines thermischen Fehlers ein Verhältnis aus einem Gas-Summenparameter ungesättigter Kohlenwasserstoffe und aus einem Gas-Summenparameter gesättigter Kohlenwasserstoffe gebildet wird.

3. Verfahren nach einem der vorhergehenden Ansprüche, wobei zusätzlich mindestens ein Einzelgasparameter bestimmt wird.

4. Verfahren nach Anspruch 3, wobei zur Bestimmung eines thermischen Fehlers ein Verhältnis aus einem Gas-Summenparameter gesättigter Kohlenwasserstoffe und einem Einzelgasparameter gebildet wird.

5. Verfahren nach einem der Ansprüche 3 oder 4, wobei zur Bestimmung eines elektrischen Fehlers ein Verhältnis aus Acetylen und einem Gas-Summenparameter gesättigter Kohlenwasserstoffe gebildet wird.

6. Verfahren nach einem der Ansprüche 3 bis 5, wobei zur Bestimmung eines elektrischen Fehlers ein Gas-Summenparameter ungesättigter Kohlenwasserstoffe zuzüglich eines Quotienten aus Acetylen und einem Gas-Summenparameter gesättigter Kohlenwasserstoffe gebildet wird.

7. Verfahren nach einem der vorhergehenden Ansprüche, wobei zur Bestimmung eines Zustands einer festen Isolierung, insbesondere eines Zelluloseabbaus, zusätzlich ein Quotient aus Kohlendioxid und Kohlenmonoxid gebildet wird.

8. Verfahren nach einem der vorhergehenden Ansprüche, wobei zum Messen des mindestens einen Gas-Summenparameters eine Infrarot-Messeinrichtung (9) mit mindestens einem Infrarotsensor verwendet wird.

9. Verfahren nach einem der vorhergehenden Ansprüche, wobei als die Isolierflüssigkeit Öl (4) verwendet wird.

10. Isolierflüssigkeits-Bestimmvorrichtung (1, 11) für einen Transformator (13), welche einen Extraktor (1) und eine Auswerteeinheit (11) aufweist , wobei
- der Extraktor (1) eine Isolierflüssigkeitsseite (8) und eine Gasseite (7) aufweist und zum Extrahieren von mehreren Einzelgasen (5) aus der Isolierflüssigkeitsseite (8) in die Gasseite (7) eingerichtet ist,
- die Gasseite (7) des Extraktors (1) mindestens eine Infrarot-Messeinrichtung (9) mit mindestens einem Infrarotsensor zum Bestimmen mindestens des Gases in der Gasseite aufweist und
- die Auswerteeinheit (11) ausgelegt ist zum Bestimmen mindestens eines Gasparameters der extrahierten Einzelgase (5),
**dadurch gekennzeichnet, dass**
die Isolierflüssigkeits-Bestimmvorrichtung (1, 11) zum Durchführen eines Verfahrens nach einem der vorhergehenden Ansprüche eingerichtet ist und die Auswerteeinheit (11) zum Bestimmen des mindestens einen Gas-Summenparameters ausgebildet ist.

11. Isolierflüssigkeits-Bestimmvorrichtung (1, 11) nach Anspruch 10, wobei die Auswerteeinheit (11) einen Prozessor zum Durchführen eines Analysealgorithmus aufweist.

12. Transformator (13), aufweisend eine Isolierflüssigkeits-Bestimmvorrichtung, **dadurch gekennzeichnet, dass** die Isolierflüssigkeits-Bestimmvorrichtung eine Isolierflüssigkeits-Bestimmvorrichtung (1, 11) nach einem der Ansprüche 10 oder 11 ist.

## Claims

1. Method for determining at least one gas parameter for a transformer, in which a number of individual gases (5) are extracted from an insulating liquid (4) and the at least one gas parameter is determined by means of an evaluation unit (11) from a measuring signal and/or from a value derived therefrom,
**characterized in that**
the at least one gas parameter comprises at least one gas sum parameter that is formed for a group of unsaturated hydrocarbons and/or at least one gas sum parameter that is formed for a group of saturated hydrocarbons.

2. Method according to Claim 1, a ratio of a gas sum parameter of unsaturated hydrocarbons and a gas sum parameter of saturated hydrocarbons being formed for determining a thermal fault.

3. Method according to one of the preceding claims, at least one individual gas parameter being additionally determined.

4. Method according to Claim 3, a ratio of a gas sum parameter of saturated hydrocarbons and an individual gas parameter being formed for determining a thermal fault.

5. Method according to either of Claims 3 and 4, a ratio of acetylene and a gas sum parameter of saturated hydrocarbons being formed for determining an electrical fault.

6. Method according to one of Claims 3 to 5, a gas sum parameter of unsaturated hydrocarbons plus a quotient of acetylene and a gas sum parameter of saturated hydrocarbons being formed for determining an electrical fault.

7. Method according to one of the preceding claims, a quotient of carbon dioxide and carbon monoxide being additionally formed for determining a state of a fixed insulation, in particular a cellulose degradation.

8. Method according to one of the preceding claims, an infrared measuring device (9) with at least one infrared sensor being used for measuring the at least one gas sum parameter.

9. Method according to one of the preceding claims, oil (4) being used as the insulating liquid.

10. Insulating liquid determining device (1, 11) for a transformer (13), which has an extractor (1) and an evaluation unit (11),
- the extractor (1) having an insulating liquid side (8) and a gas side (7) and being designed for extracting a number of individual gases (5) from the insulating liquid side (8) into the gas side (7),
- the gas side (7) of the extractor (1) having at least one infrared measuring device (9) with at least one infrared sensor for determining at least the gas in the gas side and
- the evaluation unit (11) being designed for determining at least one gas parameter of the extracted individual gases (5),
**characterized in that**
the insulating liquid determining device (1, 11) is designed for carrying out a method according to one of the preceding claims and the evaluation unit is designed for determining the at least one gas sum parameter.

11. Insulating liquid determining device (1, 11) according to Claim 10, the evaluation unit (11) having a processor for carrying out an analysis algorithm.

12. Transformer (13), having an insulating liquid determining device, **characterized in that** the insulating liquid determining device is an insulating liquid determining device (1, 11) according to either of Claims 10 and 11.

## Revendications

1. Procédé de détermination d'au moins un paramètre d'un gaz pour un transformateur, dans lequel on extrait plusieurs gaz ( 5 ) individuels d'un liquide ( 4 ) isolant et, au moyen d'une unité ( 11 ) d'exploitation, on détermine le au moins un paramètre d'un gaz à partir d'un signal de mesure et/ou d'une valeur qui s'en déduit,
**caractérisé en ce que**
le au moins un paramètre d'un gaz comprend un paramètre pour une somme de gaz formé pour un groupe d'hydrocarbures insaturés et/ou au moins un paramètre pour une somme de gaz formé pour un groupe d'hydrocarbures saturés.

2. Procédé suivant la revendication 1, dans lequel, pour déterminer une erreur thermique, on forme un rapport entre un paramètre pour une somme de gaz d'hydrocarbures insaturés et un paramètre pour une somme de gaz d'hydrocarbures saturés.

3. Procédé suivant l'une des revendications précédentes, dans lequel on détermine, en outre, au moins un paramètre pour un gaz individuel.

4. Procédé suivant la revendication 3, dans lequel, pour déterminer une erreur thermique, on forme un rapport entre un paramètre pour une somme de gaz d'hydrocarbures saturés et un paramètre pour un gaz individuel.

5. Procédé suivant l'une des revendications 3 ou 4, dans lequel, pour déterminer une erreur électrique, on forme un rapport entre l'acétylène et un paramètre d'une somme de gaz d'hydrocarbures saturés.

6. Procédé suivant l'une des revendications 3 à 5, dans lequel, pour déterminer une erreur électrique, on forme un paramètre d'une somme de gaz d'hydrocarbures insaturés additionné d'un quotient de l'acétylène et d'un paramètre de somme de gaz d'hydrocarbures saturés.

7. Procédé suivant l'une des revendications précédentes, dans lequel, pour déterminer un état d'un isolant solide, notamment d'une décomposition de cellulose, on forme supplémentairement un quotient entre le dioxyde de carbone et le monoxyde de carbone.

8. Procédé suivant l'une des revendications précédentes, dans lequel, pour mesurer le au moins un paramètre d'une somme de gaz, on utilise un dispositif ( 9 ) de mesure par infrarouge ayant au moins une sonde d'infrarouge.

9. Procédé suivant l'une des revendications précédentes, dans lequel on utilise de l'huile ( 4 ) comme liquide isolant.

10. Dispositif ( 1, 11 ) de détermination d'un liquide isolant pour un transformateur, qui a un extracteur ( 1 ) et une unité ( 11 ) d'exploitation,
dans lequel
- l'extracteur ( 1 ) a un côté ( 8 ) de liquide isolant et un côté ( 7 ) de gaz et est conçu pour extraire plusieurs gaz ( 5 ) individuels du côté ( 8 ) du liquide isolant au côté ( 7 ) du gaz,
- le côté ( 7 ) du gaz de l'extracteur ( 1 ) a au moins un dispositif ( 9 ) de mesure par infrarouge, comprenant au moins une sonde infrarouge de détermination d'au moins le gaz du côté du gaz et
- l'unité ( 11 ) d'exploitation est conçue pour déterminer au moins un paramètre de gaz des gaz ( 5 ) individuels extraits,
**caractérisé en ce que**
le dispositif ( 1, 11 ) de détermination d'un liquide isolant est conçu pour effectuer un procédé suivant l'une des revendications précédentes et l'unité ( 11 ) d'exploitation est constituée pour la détermination du au moins un paramètre d'une somme de gaz.

11. Dispositif ( 1, 11 ) de détermination d'un liquide isolant suivant la revendication 10, dans lequel l'unité ( 11 ) d'exploitation est un processeur pour effectuer un algorithme d'analyse.

12. Transformateur ( 13 ) ayant un dispositif de détermination d'un liquide isolant, **caractérisé en ce que** le dispositif de détermination d'un liquide isolant est un dispositif ( 1, 11 ) de détermination d'un liquide isolant suivant l'une des revendications 10 ou 11.
